# EUROPÄISCHE PATENTSCHRIFT

(11) **EP 1 805 502 B1**
(45) Veröffentlichungstag und Bekanntmachung des Hinweises auf die Patenterteilung: **15.04.2015**
(21) Anmeldenummer: 05797776.1
(22) Anmeldetag: 19.10.2005
(51) Int. Cl.: G01N 21/45, G01N 33/542, G01N 33/543

(54) **VERFAHREN ZUR UNTERSUCHUNG BIOCHEMISCHER WECHSELWIRKUNGEN**
METHOD FOR EXAMINING BIOCHEMICAL INTERACTIONS
PROCEDE D'EXAMEN D'INTERACTIONS BIOCHIMIQUES

(30) Priorität: 19.10.2004 DE 102004051098; 31.03.2005 DE 102005015030
(43) Veröffentlichungstag der Anmeldung: 11.07.2007
(73) Patentinhaber: Biametrics GmbH, 72076 Tübingen (DE)
(72) Erfinder: FRANK, Rüdiger, 72401 Haigerloch (DE); MÖHRLE, Bernd, 72076 Tübingen (DE); GAUGLITZ, Günter, 72070 Tübingen (DE)
(74) Vertreter: Wüstefeld, Regine Marie
(86) Internationale Anmeldenummer: PCT/EP2005/011218
(87) Internationale Veröffentlichungsnummer: WO 2006/042746

(56) Entgegenhaltungen:
- DE-A1- 3 832 185
- DE-A1- 4 200 088
- DE-A1- 19 830 727
- US-A1- 2004 070 764

## Beschreibung

Die vorliegende Erfindung betrifft ein Verfahren zur zeitaufgelösten markierungsfreien Untersuchung biochemischer Reaktionen, Bindungs- und Anlagerungsvorgängen in oder an einer dünnen Schicht.

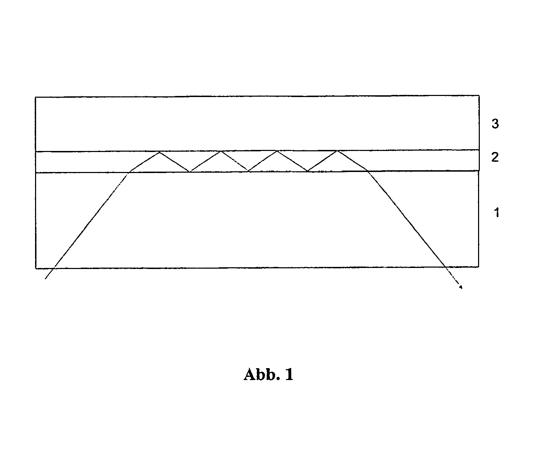

Dem in der vorliegenden Erfindung beschriebenen Verfahren liegt ein sogenannter Effekt der Optik dünner Schichten zugrunde. Vereinfacht läßt sich der verwendete Effekt durch die diesen Stand der Technik wiedergebende Abbildung 1 darstellen. In diesem Fall liegt als Schichtsystem ein Träger 1 (z. B. aus Glas, aus einem speziell beschichteten Glas oder aus Kunststoff), eine darauf aufgetragene dünne Schicht 2 (die "selektive" Schicht, an der der Effekt gemessen wird) und ein an diese Schicht angrenzendes optisches Medium 3 (z. B. eine Flüssigkeit oder ein Gas) vor. Der beobachtete Effekt ist eine von der Wellenlänge des Lichts und der Dicke der (selektiven) dünnen Schicht abhängige Intensität des reflektierten Lichts.

Ein Verfahren zum Nachweis physikalischer, chemischer und/oder biochemischer Reaktionen und Wechselwirkungen basierend auf dem gleichen Prinzip ist z. B. aus DE 198 30 727 bekannt. Bei diesem Verfahren werden mit RIfS (Reflektometrische Interferenzspektroskopie) markierungsfreie optische Bindungsvorgänge mit qualitativ gutem Ergebnis beobachtet. Allerdings müssen hierzu spezielle optisch vergütete und damit teure Träger benutzt werden. Außerdem ist das Verfahren relativ aufwendig in der Handhabung und läßt eine Parallelisierung der Messung sowie eine Miniaturisierung des Meßaufbaus nur eingeschränkt zu.

Mit der DE 42 00 088 A1 wurden erstmals ein Verfahren und eine Vorrichtung zum Nachweis physikalisch-chemischer und biochemischer Wechselwirkungen beschrieben, die auf der zuvor erläuterten reflektometrischen Interterenzspektroskopie (RIfS) beruhen und markierungsfreie optische Bindungsvorgänge ebenfalls mit qualitativ gutem Ergebnis nachweisbar machten. Bei dem in diesem Dokument offenbarten Verfahren wird die Änderung der Transmission oder Reflexion noch über die spektrale Verschiebung des gesamten Transmissions- bzw. Reflexionsspektrums gemessen. Nachteilig bei dieser grundlegenden Arbeit war der enorme Zeitaufwand, welcher erforderlich war, um die anhand der Meßwerte erhaltene, enorm hohe Datenmenge auszuwerten und der Änderung der optischen Schichtdicke zuzuordnen.

Mit SPR (Surface Plasmon Resonance) werden im Prinzip die gleichen Vorgänge beobachtet, allerdings müssen die Träger aufgrund des physikalischen Funktionsprinzips mit einem Metallfilm beschichtet werden. In den allermeisten Fällen wird dabei Gold oder Silber verwendet (siehe Nylander et, al, Sens. Actuators B 3 (1982) 79-88; Liedberg et. AI., Sens. Actuators B 4 (1983) 299-304; Homola et. AI., Sens. Actuators B 54 (1999) 3-15; Melendez et. al., Sens. Actuators B 38/39 (1997) 375-379).

Bekannte integrierte optische Methoden wie das Mach-Zehnder- oder Young-Interferometer sind technisch sehr aufwendig und teuer, da die Träger mit Wellenleiterstrukturen versehen werden müssen (siehe J. Ingenhoff et al., Fresenius J. Anal. Chem., 346 (1993) 580; B. Drapp et. al., Sensors and Actuators B 38-39 (1997) 277; P.V. Lambeck et. al., Medical and Biological Engeneering and Computing, Vol. 34, Suppl. 1 (1996), 145-148; A. Brandenburg et. al., O.S. Wolfbeis, ed., Proc. SPIE 1510, (1991) 148-159).

Des weiteren wird in der US 2004/0070764 A1 ein biochemischer Sensor beschrieben, der Interferenzen an dünnen Schichten, bedingt durch das Binden von biochemischen Substanzen an der dünnen Schicht, mit hohen Durchsatzraten messen soll. Dabei macht sich dieses Verfahren die Tatsache zunutze, daß durch die Interferenz an dünnen Schichten mittels monochromatischem Licht Interferenzfarben entstehen, die in Reflexion gemessen werden können, um so z.B. die Interaktion zwischen einem Protein und einem entsprechenden Antikörper sichtbar und detektierbar zu machen. Die Reaktion wird dabei als Reflexionsabschwächung über einen längeren Wellenlängenbereich verfolgt und detektiert.

Eine interferometrische Anordnung zur Erfassung von Feuchtigkeit als Feuchtesensor ist in der DE 38 32 185 A1 beschrieben. Diese Anordnung besteht aus zumindest einer dünnen Schicht mit wenigstens einer lichtdurchlässigen Schicht aus einem Material, das einen feuchteabhängigen Brechungsindex aufweist. Der Brechungsindex und damit die Feuchte wird über eine Messung der Reflektivität oder Transmissivität bestimmt.

Für einen Nachweis markierungsfreier optischer Bindungsvorgänge ist diese Anordnung nicht geeignet, da bei der Herstellung des Sensors eine Wellenlänge als sogenannte Designwellenlänge festgelegt und danach die optische Dicke der jeweils verwendeten Schicht(en) bemessen wird, während das Verfahren zum Nachweis von biochemischen Wechselwirkungen mittels der reflektometrischen Interferenzspektroskopie (RIfS) darauf beruht, diese Wellenlänge erst zu ermitteln.

Der Erfindung liegt somit die Aufgabe zugrunde, ein Verfahren zum Nachweis biochemischer Reaktionen, Bindungs- und Anlagerungsvorgänge in oder an dünnen Schichten bereitzustellen, bei dem die Meßergebnisse weitgehend unabhängig vom Trägermaterial sind, das eine Parallelisierung der Messung und eine Miniaturisierung des Meßaufbaus ermöglicht, und bei dem die Meßergebnisse wesentlich schneller und einfacher als bei bekannten Verfahren, praktisch on-line, ausgewertet werden können.

Diese Aufgabe wird durch das Verfahren mit den Merkmalen des Anspruches 1 gelöst. Bevorzugte Ausführungsformen des Verfahrens sind in den abhängigen Ansprüchen 2 bis 8 dargestellt. Der Wortlaut sämtlicher Ansprüche wird hiermit durch Bezugnahme zum Inhalt dieser Beschreibung gemacht.

Erfindungsgemäß wird diese Aufgabe dadurch gelöst, daß die bisher bekannte, auf Reflexionsmessungen basierende Detektion von einem Spektrum, bestehend aus mehreren Wellenlängen, auf die Detektion nur einer einzigen optimalen Wellenlänge reduziert wird.

Praktisch wird dies dadurch erreicht, daß die dünne Schicht mittels einer Lichtquelle mit Licht bestrahlt und der an der dünnen Schicht reflektierte Strahlungsanteil an einem Detektor gemessen wird. Dabei sind die Anordnung und die Beschaffenheit der Lichtquelle und gegebenenfalls des mindestens einen zwischen der Lichtquelle und dem Detektor positionierten Monochromators so gewählt, daß am Detektor monochromatisches Licht einer einziges Wellenlänge (oder eines sehr schmalen Spektrums) ankommt. Dies kann z. B. dadurch erzielt werden, daß die verwendete Lichtquelle gleich monochromatisches Licht ausstrahlt, so daß auf die Verwendung von Monochromatoren verzichtet werden kann. Als monochromatische Lichtquelle können insbesondere Leuchtdioden (LED), Laserdioden oder Laser verwendet werden. Anstelle eines Monochromators kann auch jede spektral auflösende Vorrichtung, insbesondere ein Interferenzgitter oder eine Prisma verwendet werden.

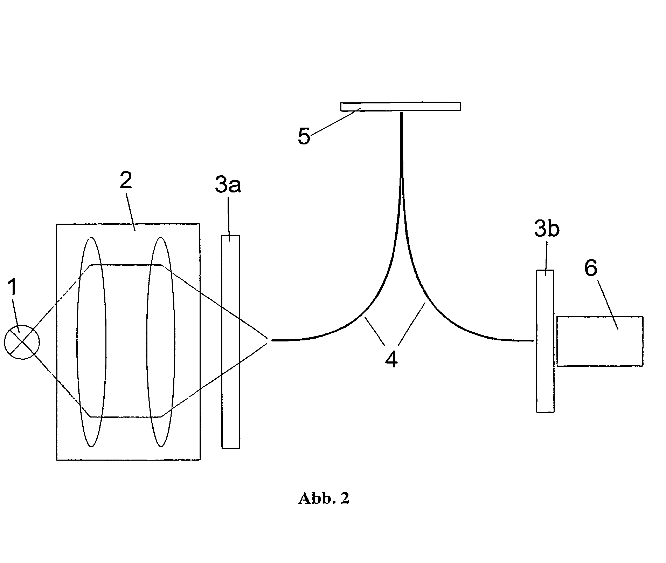

Wenn eine polychromatische Lichtquelle benutzt wird, wird ihr, dem Strahlengang folgend, mindestens ein Monochromator nachgeordnet. Der Monochromator kann in diesen Fällen wiederum entweder vor (siehe auch 3a, Abb. 2) oder hinter (siehe auch 3b, Abb. 2) der dünnen Schicht angeordnet werden. Wichtig ist hierbei, daß erfindungsgemäß am Detektor das Licht eine einzigen Wellenlänge ankommt. Unter einem Detektor wird hier eine Vorrichtung verstanden, die Licht in eine meßbare Größe, z. B. in elektrischen Strom umwandelt.

Das auf die dünne Schicht einfallende Licht kann erfindungsgemäß über Einkoppelelemente geleitet werden. Zu diesen gehören bevorzugt Linsen, Lindensysteme, Spiegel oder vorzugsweise Lichtleiter, z. B. faseroptische Wellenleiter. Ebenso können statt Einsatz von Einkoppelelementen auch entsprechende Freistrahlaufbauten realisiert werden.

Unter dem Begriff "dünne Schicht" wird in der vorliegenden Erfindung eine Schicht verstanden, deren Dicke vorzugsweise zwischen 0,1 nm und 100 µm, insbesondere zwischen 10 nm und 2.000 nm, liegt. Die dünne Schicht besteht immer aus einer sensitiven (der selektiven) Schicht, deren Dicke und/oder Dichte sich nach einer erfolgten biochemischen Reaktion oder Bindung ändert, wobei es gilt, diese Dicken- und/oder Dichteänderung zu bestimmen. Die dünne Schicht kann außer der sensitiven zusätzlich weitere Schichten beinhalten, deren Dicke sich bevorzugt durch erfolgte Reaktion, Bindung oder sonstige Wechselwirkungen nicht ändert. Diese zusätzlichen Schichten können einer besseren Haftung der sensitiven Schicht auf einem Träger dienen oder zur Koppelung mit anderen Analysemethoden oder zur Änderung der optischen Eigenschaften des Schichtsystems verwendet werden.

Die zu untersuchende dünne Schicht kann auf einem festen Träger angeordnet sein. Die Ausrichtung der dünnen Schicht auf dem Träger kann sowohl der einfallenden Strahlung zu- als auch abgewandt sein. Im zweiten Fall muß das Trägermaterial zumindest teilweise für das Licht der verwendeten Wellenlänge durchlässig sein.

Die dünne Schicht kann bei der Messung im Bezug zu einfallenden Lichtstrahlung in einem Winkel kleiner 90 ° positioniert sein. Vorzugsweise ist die dünne Schicht senkrecht (90 °) zur einfallenden Lichtstrahlung positioniert. Im Fall eines auf die dünne Schicht senkrecht einfallenden Lichtstrahls werden für eine Zu- und Ableitung des einfallenden bzw. des reflektierten Lichtes Lichtleiter benutzt.

In der Abb. 2 ist eine der bevorzugten Varianten des Meßaufbaus dargestellt, mit dem das erfindungsgemäße Verfahren durchgeführt werden kann. Es handelt sich in diesem Fall um eine Variante mit einer polychromatischen Lichtquelle 1, wobei das Licht über Einkoppelelemente 2 und eine faseroptische Lichtleitung (Lichtleiter 4) auf einen Träger 5 mit einer darauf aufgetragenen dünnen Schicht in einem rechten Winkel (90 °) zugeleitet wird und der ebenfalls im rechten Winkel (90 °) reflektierte Lichtanteil über die Lichtleiter 4 auf einen Detektor 6 abgeleitet wird. Damit am Detektor das Licht einer einzigen Wellenlänge ankommt, wird entweder zwischen der Lichtquelle und dem Träger oder zwischen dem Träger und dem Detektor ein Monochromator (3a bzw. 3b) positioniert.

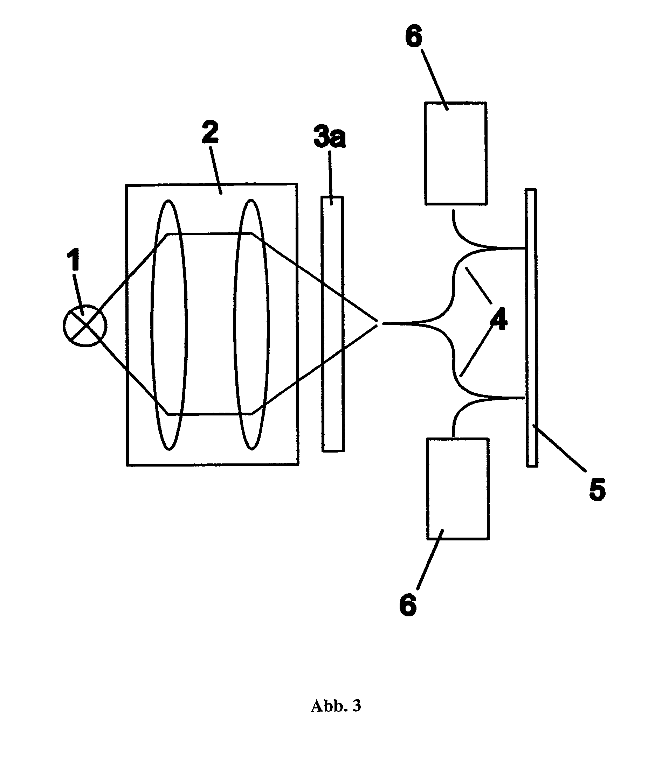

In der Abb. 3 ist eine weitere bevorzugte Variante abgebildet, bei der (ausgehend von Abb. 2) ein Zweikanal-Meßplatz unter Zuhilfenahme zweier Photodioden 6 und einer angepaßten Wellenleiteranordnung 4 realisiert werden kann. Der zweite Kanal kann z. B. zur Messung von Referenzwerten benutzt werden, die bei der Auswertung zur Ermittlung einer Intensitätsänderung direkt herangezogen werden können.

Einen besonders wichtigen Aspekt des erfindungsgemäßen Verfahrens stellt die Auswahl oder Ermittlung der für die Auswertung der Meßergebnisse optimalen Wellenlänge dar. Diese entspricht vorzugsweise einer Wellenlänge, bei der die Änderung der Lichtintensität nach einer erfolgten Reaktion oder Wechselwirkung an oder in der dünnen Schicht groß, insbesondere maximal (optimal) ist.

Die optimale Wellenlänge hängt von den Eigenschaften der dünnen Schicht, insbesondere ihrer optischen Dicke, sowie von den Eigenschaften des Trägers und seiner eventuellen zusätzlichen Beschichtung ab. Durch die bekannte Optik an dünnen Schichten entstehen in jeder dünnen Schicht Vielfachreflexionen. Unabhängig vom Schichtsystem auf dem Träger inklusive der dünnen Schicht ergibt sich in der Reflexion für jede Wellenlänge eine periodische Reflexion in Abhängigkeit der optischen Dicke der dünnen Schicht.

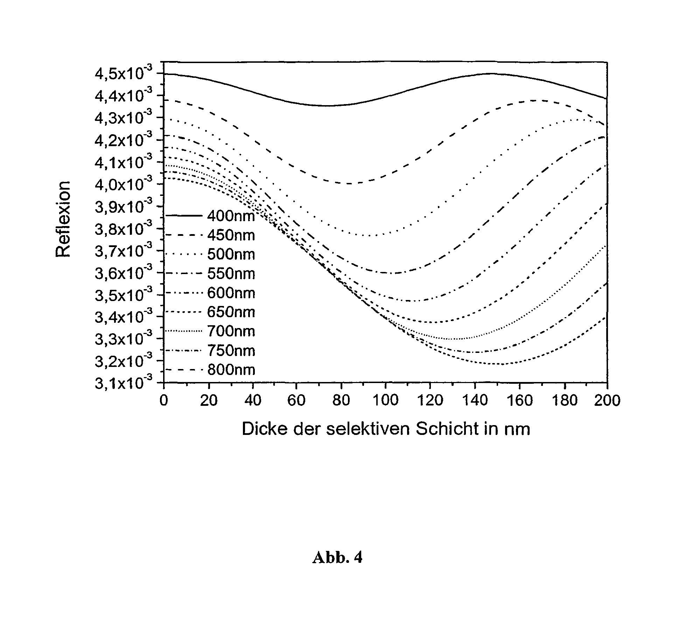

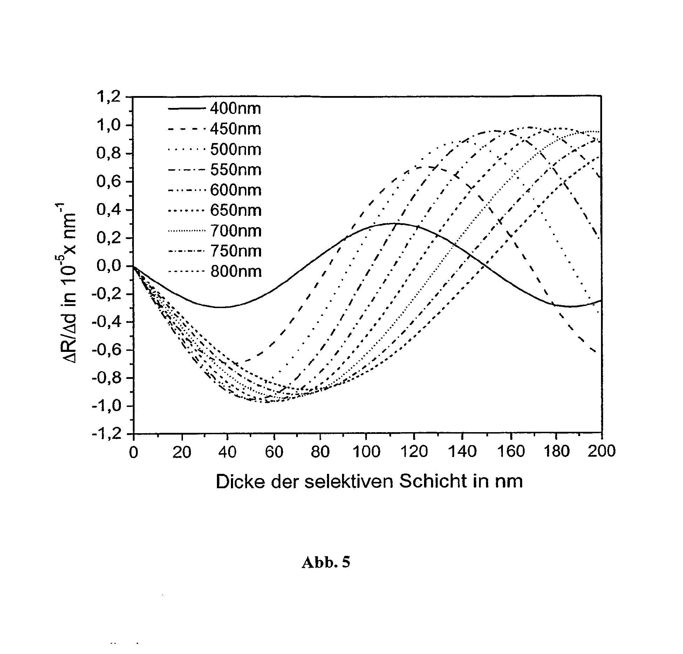

Die optimale Wellenlänge wird gemäß einer ersten Variante experimentell so bestimmt, daß sie sich im Bereich eines Wendepunktes des Dünnschichtspektrums befindet, wobei die Lichtintensität als Funktion der Wellenlänge aufgetragen wird. Dort liefert die Ableitung der Steigung der Intensität des reflektierten Lichtes nach der optischen Schichtdickenänderung der dünnen Schicht den größten Betrag und damit die größte Signaländerung. Zudem ist die Steigerung der Intensität in diesem Bereich aufgrund der überaus geringen Effekte, die zu messen sind, in sehr guter Nährung linear. Alternativ wird die Ermittlung der optimalen Wellenlänge gemäß einer zweiten Variante auch anhand der Daten über die Eigenschaften der dünnen Schicht, wie z. B. die Schichtdicke und Brechungs-index, theoretisch berechnet (Abb. 4 und 5). Die Wahl der Methode für die Ermittlung der optimalen Wellenlängen hängt davon ab, ob die Eigenschaften des Schichtsystems bekannt sind oder nicht.

Alternativ kann auch ein optimales Schichtsystem zu einer vorgegebenen Wellenlänge theoretisch ermittelt und angepaßt werden. Wie in den Ausführungsbeispielen gezeigt wird, hat die Auswahl der dünnen Schicht bzw. des Schichtsystems, insbesondere seine Dicke und das Material, einen großen Einfluß auf die Signalhöhe. Weitere Faktoren, die den Signalhub des Nutzsignals beeinflussen können und bei der Optimierung des Signals berücksichtigt werden müssen, sind beispielsweise der Einfallswinkel und die Polarisation des eingehenden Lichtes.

Die vorherige Abschätzung eines optimalen Signals einen gegebenen bzw. des theoretisch zu berechnenden Schichtsystems kann die Auswahl der zu verändernden Variablen erleichtern. Insofern ergänzen sich theoretische Simulationen mit den experimentellen Messungen. Durch eine gemeinsame Nutzung beider kann das Schichtsystem optimal ausgewählt und justiert werden.

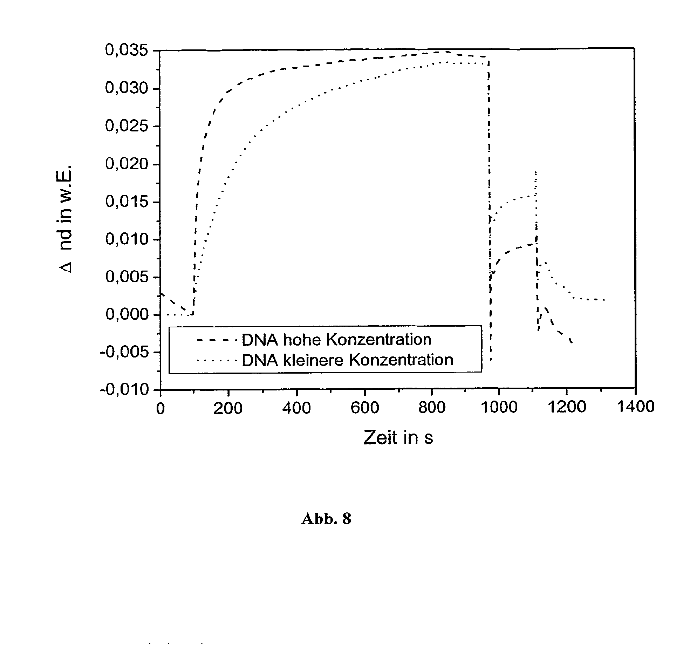

Durch die Verwendung einer einzigen optimalen Wellenlänge wird erfindungsgemäß eine weitgehende optische Unabhängigkeit vom Trägermaterial erreicht. So können bei dem erfindungsgemäßen Verfahren statt bislang notwendigen speziell vergüteten Trägern wesentlich preiswertere und in der Herstellung einfachere Materialien wie Glas, Kunststoff (z.B. TOPAS, ein Cycloolefin-Copolymer, z. B. von Ticona), beschichtetes Glas oder sonstige Materialien verwendet werden (Abb. 8 bis 10).

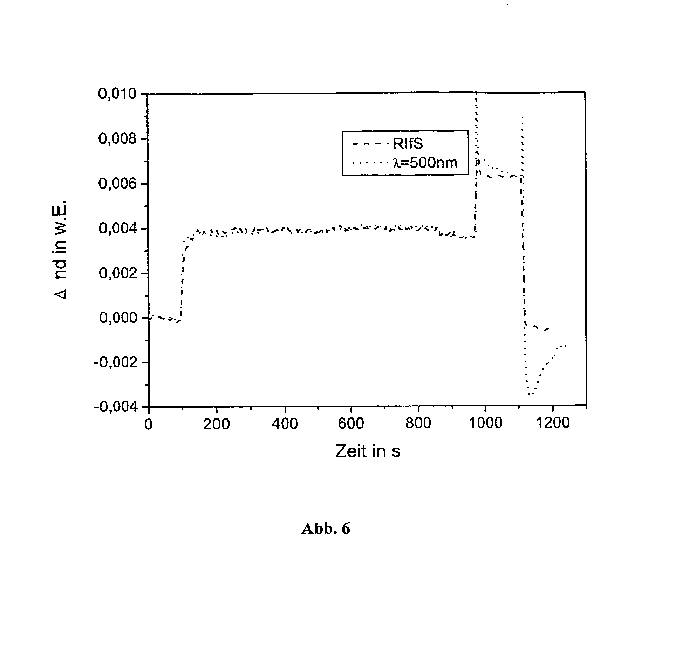

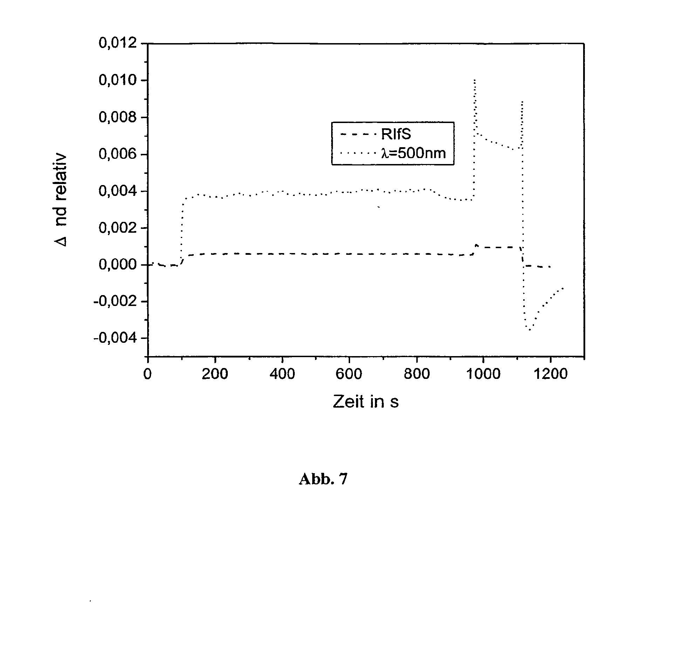

Durch die Anpassung der optimalen Wellenlänge an das jeweilige Material und die darauf befindliche Oberflächenchemie wird der Effekt der Interferenz an dünnen Schichten durch das zusätzliche Schichtwachstum an die Trägeroberfläche maximal. Dadurch wird das Signal/RauschVerhältnis optimiert. In den später erläuterten Ausführungsbeispielen wird gezeigt, daß beim Vergleich des erfindungsgemäßen Verfahrens mit dem bekannten RifS-Verfahren auf einem für RIfS notwendigen Interferenzglas das Signal/Rausch-Verhältnis um einen Faktor 5 bis 10 verbessert ist (Abb. 6 und 7).

Ein weiterer Vorteil des erfindungsgemäßen Verfahrens gegenüber bekannten Verfahren liegt darin, daß damit ein parallelisierter und miniaturisierter Aufbau mit erheblich kleinerem technischem Aufwand verbunden ist als bei bekannten Verfahren (wie RIfS). Durch eine Beschränkung der Messung auf nur eine Wellenlänge fallen die bei den herkömmlichen Verfahren notwendigen Umschaltvorgänge zwischen den verschiedenen Wellenlängen weg. Die dadurch erreichte Verkürzung der Gesamtmeßzeit kann dazu genutzt werden, die einzelnen Meßzeiten zu verlängern. Aufgrund der überaus geringen Signaländerungen trägt eine längere Meßzeit wesentlich zu einer höheren Signalgüte bei. Auch der technische Aufwand ist beim erfindungsgemäßen Verfahren um einiges geringer als bei den bekannten Methoden. Diese Faktoren machen eine Parallelisierung der Messung wesentlich einfacher als bei den bekannten Verfahren. Verringerte Meßzeit, einfache Handhabung und geringer technischer Aufwand ermöglichen auch eine einfachere technische Realisierung für eine miniaturisierte Ausführung des Prinzips.

Weitere Vorteile, Merkmale und Anwendungsmöglichkeiten der Erfindung werden nachstehend anhand der Ausführungsbeispiele mit Bezug auf die Zeichnungen beschrieben. In den Zeichnungen zeigen:
- Abb. 1:: Vereinfachtes Schichtmodell zur Veranschaulichung des der Erfindung zugrundeliegenden Prinzips. Auf dem Träger 1 befindet sich die dünne Schicht 2, die in diesem Beispiel direkt an eine Flüssigkeit 3 angrenzt. In der dünnen Schicht finden Vielfachreflexionen statt.
- Abb. 2:: Schematische Darstellung einer Anordnung zur Durchführung des erfindungsgemäßen Verfahrens mit einem Einkanal-Meßplatz. Die Vorrichtung umfaßt eine polychromatische Lichtquelle.1, die Licht im sichtbaren Bereich aussendet. Mittels einer Einkoppelvorrichtung 2 wird das Licht durch einen Monochromator 3a in einen Y-Wellenleiter 4 eingekoppelt. Der Monochromator 3a kann auch durch einen Monochromator 3b auf der anderen Seite des Y-Wellenleiters ersetzt werden. Das eingekoppelte Licht wird auf den Träger 5 mit einer darauf angeordneten zu messenden dünnen Schicht geleitet, wo es wieder in den Wellenleiter 4 zurückreflektiert wird und von einer Photodiode 6 detektiert wird. Der Monochromator kann bei Bedarf für die Auswahl des optimalen Spektralbereiches ausgewechselt werden.
- Abb. 3:: Schematische Darstellung einer Anordnung zur Durchführung des erfindungsgemäßen Verfahrens mit einem Zweikanal-Meßplatz. Dieser kann unter Zuhilfenahme zweier Photodioden 6 und einer angepaßten Wellenleiteranordnung 4 realisiert werden Der zweite Kanal kann z. B. zur Referenzmessung benutzt werden.
- Abb. 4:: Reflexion in Abhängigkeit von der Schichtdicke. Als Modell wurde hier BK7-Glas (ein bekanntes Qualitätsglas von Schott, Deutschland) als Trägermaterial, eine (hypothetische) selektive Schicht mit Brechungsindex n=1,34 und Wasser als Deckschicht angenommen. Die Dicke der selektiven Schicht wird auf der x-Achse variiert, auf der y-Achse ist der Reflexionsgrad aufgetragen.
- Abb. 5:: Ableitung der Reflexion R in Abhängigkeit der Dicke d der selektiven Schicht. Für die jeweilige Schichtdicke der selektiven Schicht wird die Wellenlänge ausgesucht, die an dieser Stelle möglichst ein Extremum hat. Aufgrund der überaus geringen Schichtdickenänderungen, die maximal nur wenige nm beträgt, ist in sehr guter Nährung eine lineare Beziehung von Reflexion zu Dickenänderung gegeben.
- Abb. 6:: Vergleich des herkömmlichen RIfS-Verfahrens mit dem erfindungsgemäßen Verfahren anhand einer Messung mit beliebiger DNA auf einem sogenannten Interferenzglas (Borosilicatglas D263 von Schott, Deutschland, Dicke 1 mm, beschichtet mit 10 nm Ta₂O₅ und 330 nm SiO₂). Die beiden Messungen wurden mit dem gleichen Chip hintereinander gemacht. Die Skalierungen in der y-Achse (w. E. = willkürliche Einheiten) wurden angepaßt, damit die beiden Bindungskurven rein optisch verglichen werden können. Der Signalhub ist mit dem erfindungsgemäßen Verfahren um einen Faktor 5 besser.
- Abb. 7:: Gleiche Messung wie in Abb. 6, nur wurden hier die relativen Signaländerungen nicht angeglichen. Der Signalhub ist mit dem erfindungsgemäßen Verfahren um einen Faktor 5 bis 10 besser.
- Abb.8:: Erfindungsgemäße Messungen an einem Kunststoff-Träger aus TOPAS (d = ca. 1 mm) mit zwei verschiedenen DNA-Konzentrationen (FBMG1, Firma IBA Göttingen). Die ausgesuchte Wellenlänge war hier 650 nm.
- Abb.9:: Bindungskurve von Anti-ACA auf Kunststoff (TOPAS s. o.) (ACA = 4-Chlor-6-(isopropylamino)-1,3,5-triazin-2-(6'-amino)capronsäure.
- Abb. 10:: Bindungskurve von Anti-ACA auf Kunststoff (TOPAS s. o.) mit niedriger Konzentration und gehemmte Bindung mit Atrazin (chemisches Pflanzenbehandlungsmittel).
- Abb. 11:: Bindungskurve von 10µg/ml Anti-ACA auf einem mit 180 nm Indium-Zinnoxid beschichteten BK7-Träger (d = ca. 1 mm) und eine gehemmte Bindung mit 1000 ppb Atrazin.
- Abb. 12:: Bindungskurve von 1µg/ml Anti-ACA auf einem D263-Substrat (d = ca. 1 mm) mit einer 10 nm dicken Ta₂O₅ und 330 nm dikken SiO₂-Schicht (s. o. Interferenzglas). Zusätzlich ist eine Meßkurve mit Ovalbumin (OVA) abgebildet, um einen eventuellen Anteil unspezifischer Bindung an die Sensoroberfläche abschätzen zu können.
- Abb. 13:: Bindungskurve von 1 µg/ml Anti-ACA auf einem D263-Substrat mit einer 45 nm dicken Ta₂O₅ und 20 nm dicken SiO₂-Schicht. Zusätzlich ist eine Meßkurve mit Ovalbumin (OVA) abgebildet, um einen eventuellen Anteil unspezifischer Bindung an die Sensoroberfläche abschätzen zu können.
- Abb. 14:: Bindungskurve von 1µg/ml Anti-ACA auf einem transparenten Kunststoffsubstrat (TOPAS s. o.). Zusätzlich ist eine Meßkurve mit Ovalbumin (OVA) abgebildet, um einen eventuellen Anteil unspezifischer Bindung an die Sensoroberfläche abschätzen zu können.
- Abb. 15:: Ableitung der Reflektivität in Abhängigkeit der Dicke der selektiven Schicht für ein D263-Substrat mit einer 10 nm dicken Ta₂O₅ und 330 nm dicken SiO₂-Schicht.
- Abb. 16:: Ableitung der Reflektivität in Abhängigkeit der Dicke der selektiven Schicht für ein D263-Substrat mit einer 45 nm dicken Ta₂O₅ und 20 nm dicken SiO₂-Schicht.
- Abb. 17:: Ableitung der Reflektivität in Abhängigkeit der Dicke der selektiven Schicht und des Einfallswinkels für ein transparentes Kunststoffsubstrat (TOPAS s. o.) bei einer Wellenlänge von 500 nm.
- Abb. 18:: Ableitung der Reflektivität in Abhängigkeit der Dicke der selektiven Schicht und des Einfallswinkels für ein D263-Substrat mit einer 10 nm dicken Ta₂O₅ und 330 nm dicken SiO₂-Schicht bei einer Wellenlänge von 500 nm.
- Abb. 19:: Ableitung der Reflektivität in Abhängigkeit der Dicke der selektiven Schicht und des Einfallswinkels für ein D263-Substrat mit einer 45 nm dicken Ta₂O₅ und 20 nm dicken SiO₂-Schicht bei einer Wellenlänge von 500nm.

### Ausführungsbeispiele

Es wurden Messungen mit dem erfindungsgemäßen Verfahren im Vergleich mit der bekannten und etablierten RIfS-Methode mit sehr guter Übereinstimmung vorgenommen. Zur Überprüfung des erfindungsgemäßen Verfahrens wurden unterschiedliche Antigen-Antikörper- sowie DNA-DNA-Wechselwirkungen gemessen. Außerdem wurden verschiedene Träger aus Glas sowie verschiedene modifizierte Träger aus Kunststoff vermessen und verglichen.

### Beispiel 1

Vergleichsmessungen von erfindungsgemäßen Verfahren mit dem RIfS-Verfahren am selben Träger aus Interferenzglas (Substrat aus D263-Glas, 10 nm Ta₂O₅ und 330 nm SiO₂). Der Träger wurde mit PEG (Polyethylenglykol) beschichtet und danach mit einer DNA als sensitive Schicht funktionalisiert. Die Messungen wurden mit einer hochkonzentrierten DNA-PBS-Lösung (PBS = phosphate buffered saline) durchgeführt, so daß eine diffusionslimitierte Bindungskinetik beobachtet werden kann (siehe Abb. 6 + 7).

### Beispiel 2

Vergleich von zwei DNA-DNA-Messungen auf einem Kunststoff-Träger. Der Träger wurde mit Aminodextran beschichtet und danach mit DNA funktionalisiert. Die Messungen wurden mit unterschiedlich hohen DNA-Konzentrationen in einer PBS-Lösung durchgeführt. Man kann die daraus resultierende unterschiedliche Bindungskinetik erkennen: bei der niedrigeren DNA-Konzentration verläuft die Bindungskurve weniger steil (siehe Abb. 8).

### Beispiel 3

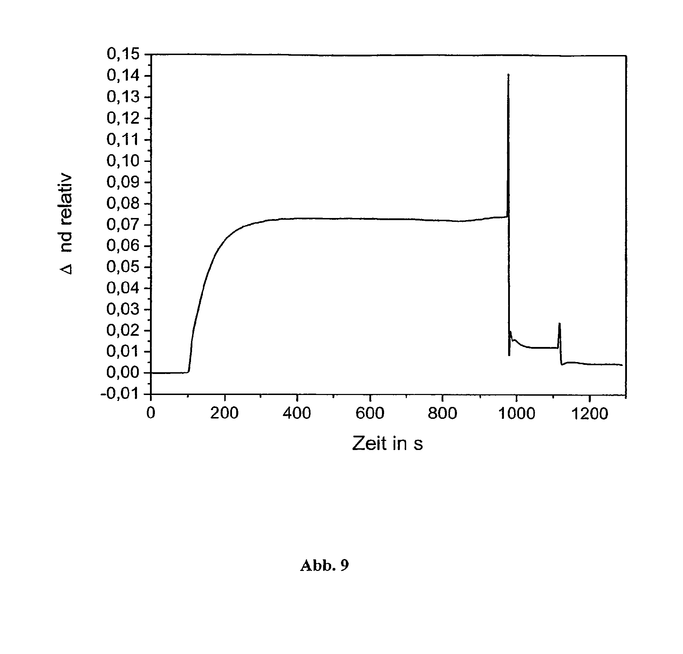

Antigen-Antikörper-Messung auf einem Kunststoff-Träger (TOPAS). Der Träger wurde mit Aminodextran beschichtet und danach mit Atrazin funktionalisiert. Die Messung wurde mit einer hoch konzentrierten Anti-ACA-PBS-Lösung durchgeführt, so daß eine diffusionslimitierte Bindungskinetik beobachtet werden kann (siehe Abb. 9).

### Beispiel 4

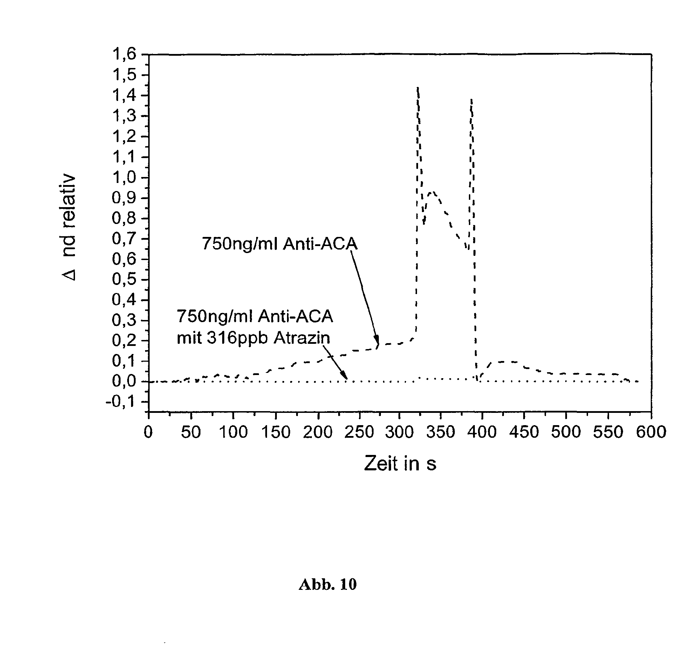

Antigen-Antikörper-Messung auf einem Kunststoff-Träger (TOPAS). Der Träger wurde mit Aminodextran beschichtet und danach mit Atrazin funktionalisiert. Die Messung wurde mit einer niedrig konzentrierten Anti-ACA-PBS-Lösung durchgeführt, so daß eine massentransportlimitierte Bindungskinetik beobachtet werden kann. Danach wurde eine mit Atrazin gehemmte Anti-ACA-PBS-Lösung vermessen, um sicherzugehen, daß die vorherigen Signaländerungen tatsächlich von der Antigen-Antikörper-Reaktion herrühren (siehe Abb. 10).

### Beispiel 5

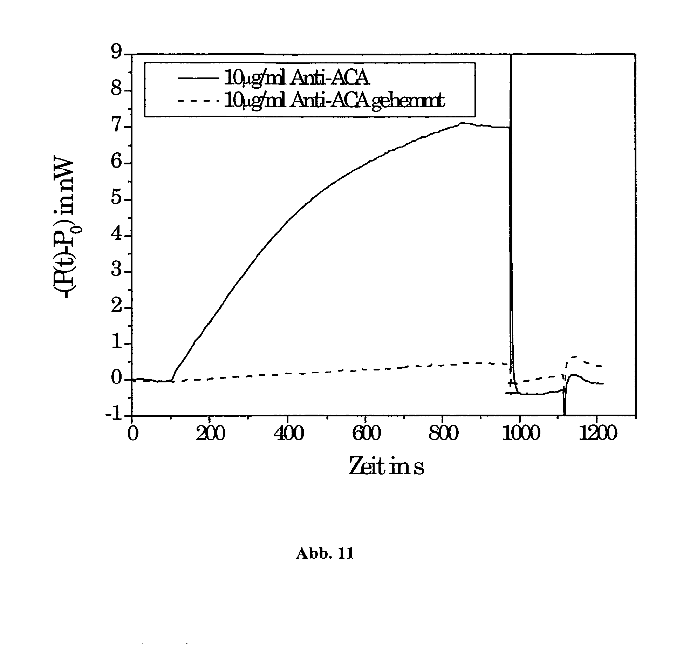

Antigen-Antikörper-Messung auf einem mit 180 nm Indium-Zinnoxid beschichteten BK7-Träger. Der Träger wurde mit Aminodextran beschichtet und danach mit Atrazin funktionalisiert. Die Messung wurde mit einer Anti-ACA-PBS-Lösung mit einer Anti-ACA-Konzentration von 10 µg/ml durchgeführt (siehe Abb. 11).

### Beispiel 6

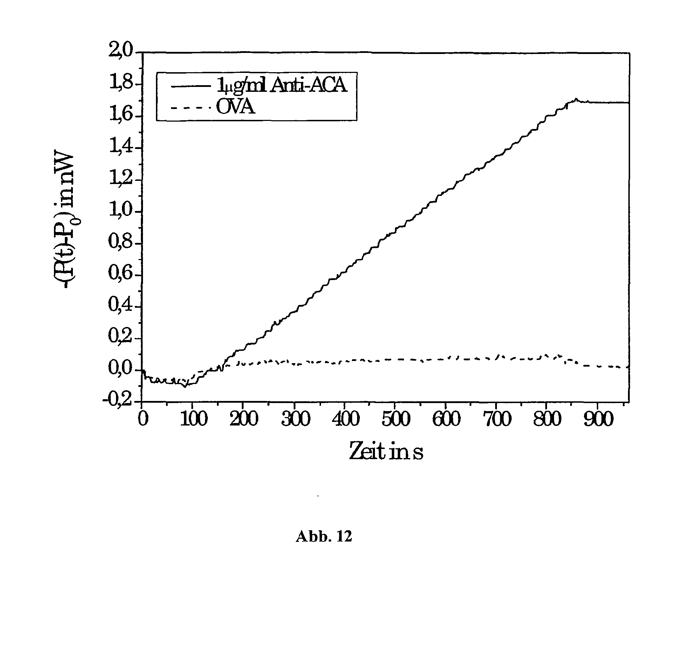

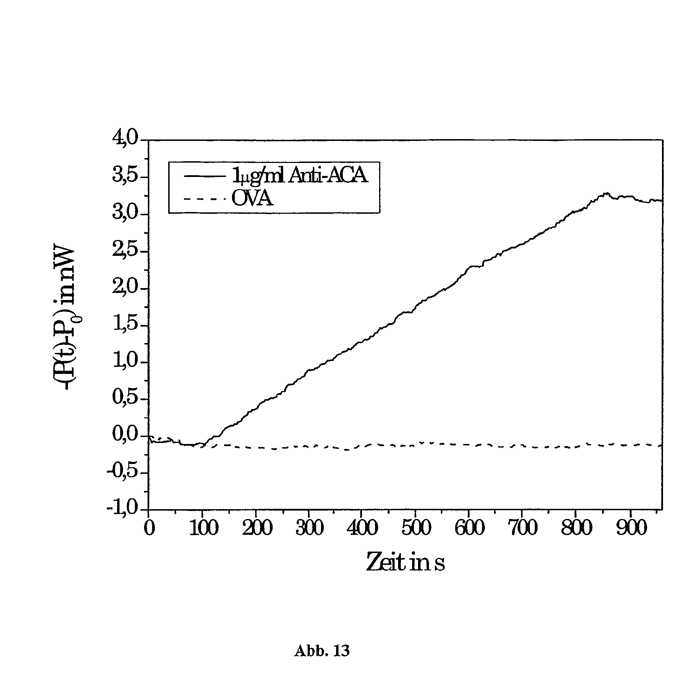

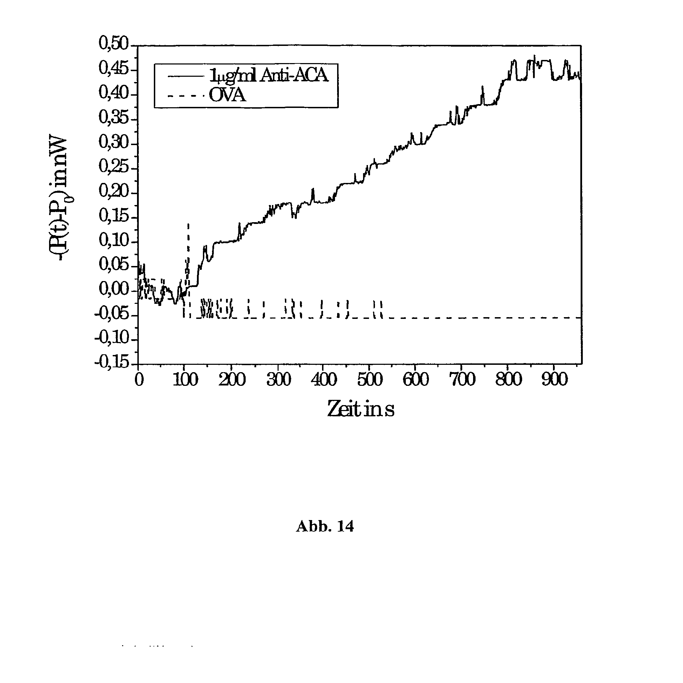

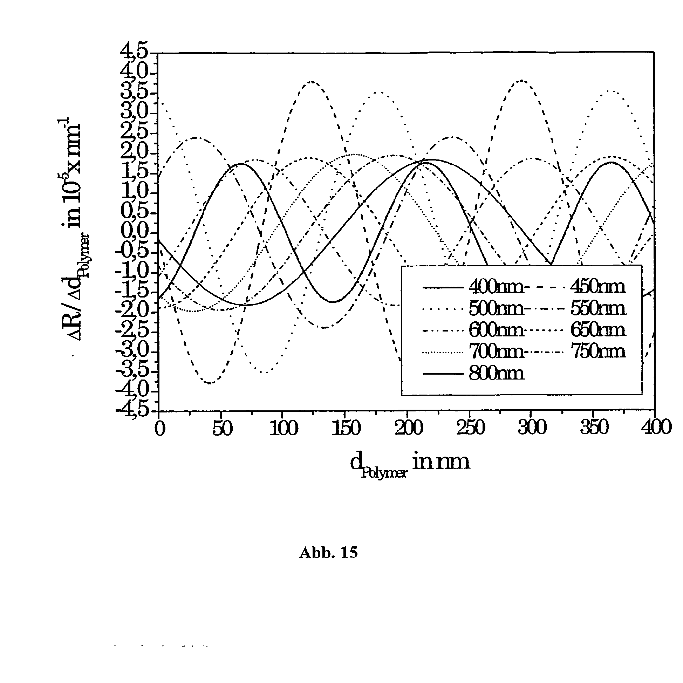

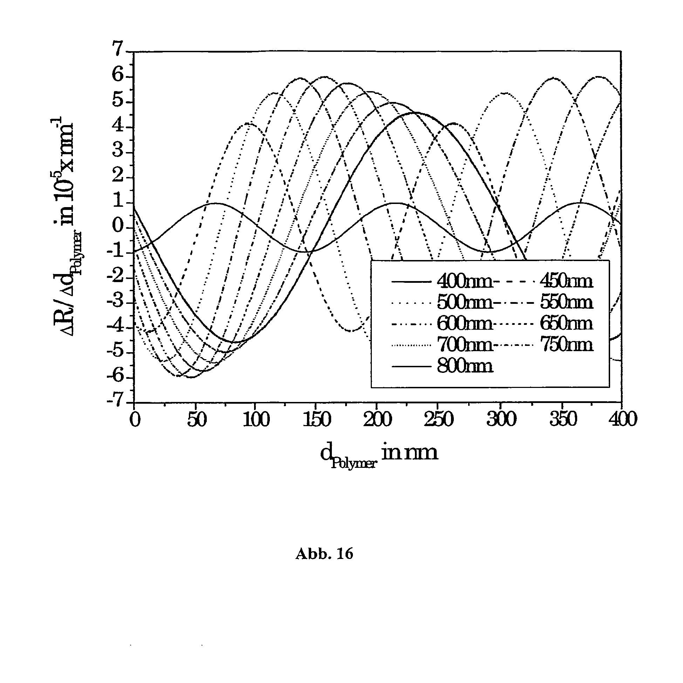

Es wird untersucht, inwiefern die Wahl des Trägermaterials bzw. dessen Beschichtung einen Einfluß auf die Signaldynamik, d. h. die Höhe der Reflektivitätsänderung bei einer optischen Schichtdickenänderung hat. Um diese ausschließlich von der Wahl des optischen Trägersystems abhängige Größe experimentell zu bestimmen, wird im Beispiel wieder das Antigen/Antikörper System Atrazin/Anti-ACA benutzt. Die Träger wurden mit Aminodextran beschichtet und danach mit Atrazin funktionalisiert. Es werden bei den Messungen aller hier untersuchten Trägersysteme die gleichen Meßprotokolle verwendet. Bei allen Messungen wurde eine Anti-ACA-PBS-Lösung mit einer Anti-ACA-Konzentration von 1µg/ml verwendet (siehe Abb. 12, 13 und 14). Bei dieser Konzentration wird eine massentransportlimitierte Bindungskinetik gewährleistet. Das bedeutet, daß alle Analytmoleküle, die im Fluß durch die Diffusionsgrenzschicht an die Sensoroberfläche gelangen, auch dort gebunden werden. Da bei allen Trägern neben den gleichen Antikörpern dieselbe Fluidik und das gleiche Flußprotokoll verwendet wurde, kann man durchaus annehmen, daß ziemlich genau jeweils die gleiche Anzahl von Antikörpern an die Sensoroberfläche anbindet und dadurch die gleiche Änderung der optischen Schichtdicke hervorruft. Der unterschiedliche Signalhub bei den einzelnen Trägern ist deshalb in sehr hohem Maße allein von den optischen Parametern des Trägersystems abhängig. Diese unterschiedliche Signaldynamik in Abhängigkeit des Trägersystems läßt sich theoretisch auch qualitativ durch unterschiedlich hohe Ableitungen der Reflexion nach der Dicke der selektiven Schicht bei unterschiedlichen Trägersystemen erklären (siehe Abb. 5, 15 und 16) mit einem einfachen Schichtmodell erklären. Zur Demonstration dieser unterschiedlichen Signaldynamik wurden folgende optische Trägersysteme vermessen:
1) Substrat aus D263-Glas, 10 nm Ta₂O₅ und 330 nm SiO₂,
2) Substrat aus D263-Glas, 45 nm Ta₂O₅ und 20 nm SiO₂,
3) unbeschichteter transparenter TOPAS-Kunststoffträger (die optischen Parameter dieses Substrats stimmen gut mit denen von BK7-Glas überein).

Nur durch die Wahl eines anderen optischen Trägersystems ergibt die gleiche Änderung der optischen Schichtdicke einen bis zu einem Faktor sieben unterschiedlich hohes Signal.

### Beispiel 7

Neben einer angepaßten optischen Vergütung für eine Maximierung der Signaldynamik kann eine optimierte geometrische Anordnung in Kombination mit einer dafür angepaßten optischen Vergütung eine weitere Maximierung der Signaldynamik ergeben.

Eine größere Reflektivität an der Grenzfläche Biopolymer/Umgebungsmedium, dadurch ein insgesamt höherer Anteil der reflektierten Strahlung höherer Ordnung und damit eine größere Signaldynamik kann durch einen schrägen Einfallswinkel erreicht werden. Im Zusammenhang mit dem jeweiligen verwendeten optischen Trägersystem existiert dann auch ein optimaler Einstrahlwinkel für maximale Signaldynamik.

Es werden Simulationen von folgenden optischen Trägersystemen präsentiert:
1) Substrat aus D263-Glas, 10 nm Ta₂O₅ und 330 nm SiO₂,
2) Substrat aus D263-Glas, 45 nm Ta₂O₅ und 20 nm SiO₂,
3) unbeschichteter transparenter Kunststoffträger (TOPAS).

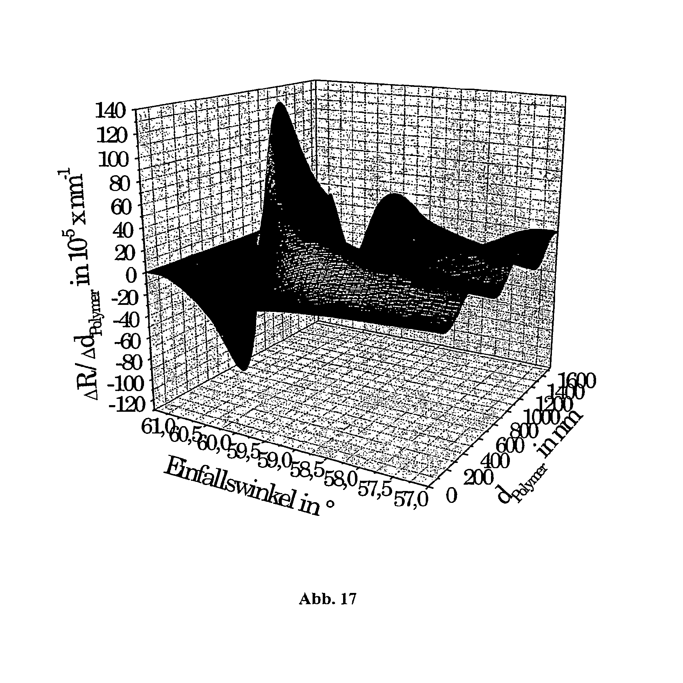

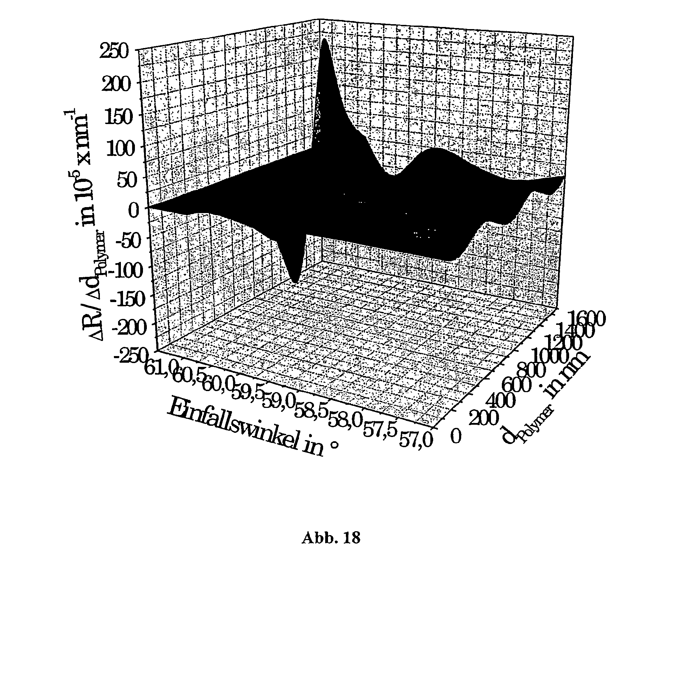

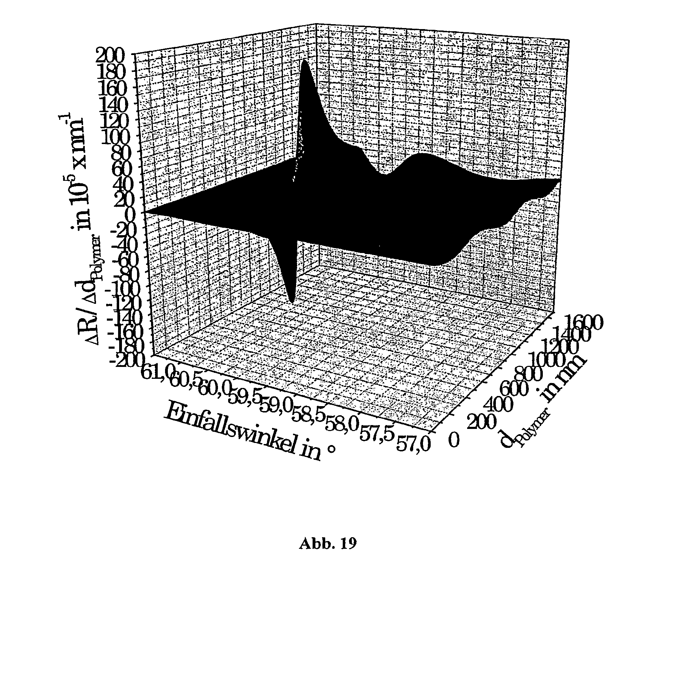

In den Abbildungen 17, 18 und 19 sind die Signaldynamiken in Abhängigkeit des Einfallswinkels und der Dicke des selektiven Biopolymers für eine Wellenlänge von 500 nm aufgetragen.

Die maximalen Signalhübe sind relativ ähnlich, die Unterschiede betragen hier zwischen den verschiedenen Trägersystemen nur ungefähr einen Faktor 2. Die Tatsache, daß die größten Signalhübe erst bei einer Polymerschichtdicke von ein paar hundert Nanometern erwartet werden würden, könnte man experimentell durch eine größere SiO₂-Schichtdicke als additive Größe zum Biopolymer kompensieren. Zu sehen ist bei allen Graphen, daß der optimale Einfallswinkel jeweils kurz vor dem Grenzwinkel zur Totalreflexion liegt. Gelingt es, diese Größen für einen geeigneten Träger experimentell optimal einzustellen, könnte der Signalhub theoretisch um ungefähr anderthalb Größenordnungen verbessert werden. Weiterhin kann bei schrägem Lichteinfall die Verwendung nur einer Polarisationsrichtung des Lichts eine zusätzliche Erhöhung der Signaldynamik bewirken.

### Beispiel 8

Für den Einsatz von portablen und miniaturisierten Sensoreinheiten wurde ein 8 x 8 mm² großer, kommerziell erhältlicher Sensor mit drei integrierten Lichtquellen in der Mitte und zwei Photodioden an der Seite verwendet. Es sind hier also schon simultane Messungen an zwei Punkten möglich. In diesem Beispiel wurde allerdings nur ein Meßpunkt detektiert. Ebenso wurde bisher auch nur eine Diode, die sogenannte Monitor-Diode, ausgelesen. Für die Messung wurde Licht einer grünen LED (light emitting diode) benutzt. Das Licht fällt schräg auf den Träger und wird dann unter dem gleichen Winkel reflektiert und von den Photodioden detektiert.

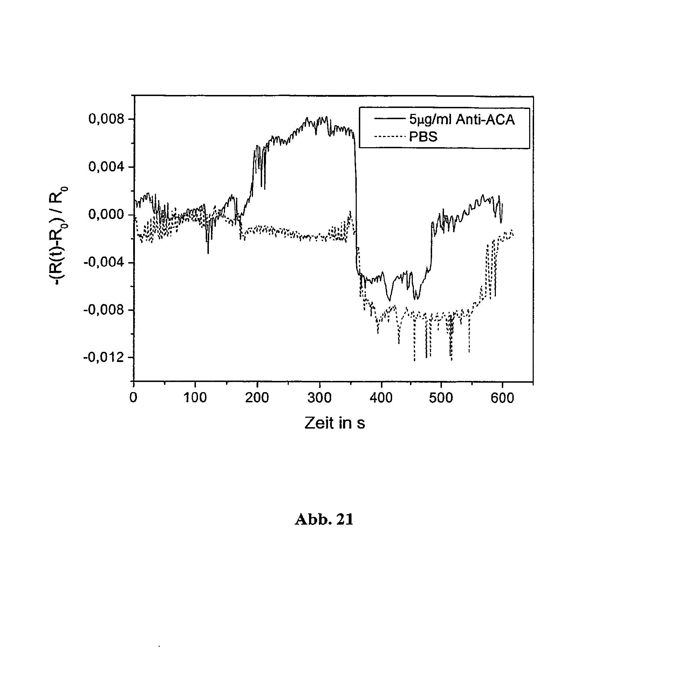

Als biologisches System wurde eine Antigen-Antikörper-Wechselwirkung auf einem Kunststoffsubstrat (TOPAS) detektiert. Dieses wurde mit Aminodextran beschichtet und danach mit Atrazin funktionalisiert. Es wurden Messungen mit einer Anti-ACA-PBS-Lösung mit einer Anti-ACA-Konzentration von 5 µg/ml durchgeführt. Um zu überprüfen, ob die detektierte Reflektivitätsänderung tatsächlich von der Anbindung der Antikörper in der Lösung an die Sensoroberfläche herrührt, wurde am gleichen Chip unter gleichen Bedingungen eine reine PBS-Lösung detektiert. Hier konnte im Vergleich zur Antikörpermessung keine signifikante Reflektivitätsänderung beobachtet werden (siehe Abb. 21).

Die Messungen zeigen die prinzipielle Eignung der Methode sowohl für den Gebrauch von Leuchtdioden als auch für eine mögliche Miniaturisierung.

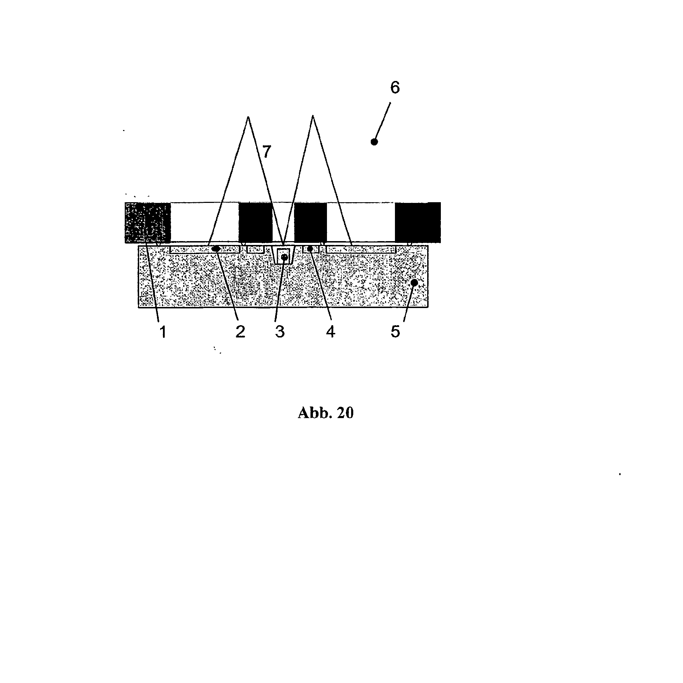

Der schematische Aufbau des Sensors für Beispiel 8 gemäß Abb. 20 zeigt eine LED 3 sowie PIN-Dioden 2 (pin = positive intrinsic negative) und Monitor-Dioden 4, die auf bzw. in ein Silizium-Substrat 5 angebracht wurden. Über diesem Silizium-Substrat befindet sich eine Glas-Keramik 1 mit oben gezeigter Struktur. Die keramischen Abschnitte haben eine Filtereigenschaft und sollen die PIN-Dioden 2 vor ungewollter Strahlung abschirmen. Über diese Glas-Keramik 1 wird nun der Träger 6 mit der selektiven Schicht aufgebracht. Für die erfindungsgemäße Methode interessante Strahlen 7 werden an der mit der selektiven Schicht beschichteten Trägerseite zur PIN-Diode 2 reflektiert und dort detektiert.

## Patentansprüche

1. Verfahren zur markierungsfreien Untersuchung biochemischer Reaktionen, Bindungs- und/oder Anlagerungsvorgänge an oder in mindestens einer dünnen Schicht, deren Dicke zwischen 0,1 nm und 100 µm, vorzugsweise zwischen 10 nm und 2.000 nm liegt, mit folgenden Schritten:
- Beleuchtung mindestens eines punktförmigen oder flächenhaften Bereichs der mindestens einen dünnen Schicht mit Licht von mindestens einer Lichtquelle (1),
- Leitung des an den hintereinander liegenden Begrenzungsflächen der mindestens einen dünnen Schicht reflektierten Strahlenanteils an mindestens einen Detektor (6),
- Detektion des an den hintereinander liegenden Begrenzungsflächen der mindestens einen dünnen Schicht reflektierten Strahlenanteils mit dem mindestens einen Detektor (6), wobei das auf den mindestens einen Detektor einfallende Licht monochromatisch ist,
- Messung der durch die Änderung der optischen Schichtdicke infolge der biochemischen Reaktionen, Bindungs- und/oder Anlagerungsvorgänge verursachten Änderung der detektierten Lichtintensität,
- Ableitung von Parametern aus der Änderung der detektierten Lichtintensität, die die zu untersuchenden Reaktionen, Bindungs- und/oder Anlagerungsvorgänge an und/oder in der mindestens einen dünnen Schicht kennzeichnen,
**dadurch gekennzeichnet, daß** für das auf den mindestens einen Detektor einfallende monochromatische Licht eine einzige optimale Wellenlänge ausgewählt wird, wobei
- die für die Untersuchung optimale Wellenlänge durch experimentelle Messungen ausgewählt wird, indem ein Dünnschichtspektrum der dünnen Schicht aufgenommen, dabei die Lichtintensität als Funktion der Wellenlänge aufgetragen und die optimale Wellenlänge im Bereich eines Wendepunktes des Dünnschichtspektrums bestimmt wird, oder
- die für die Untersuchung optimale Wellenlänge durch eine theoretische Berechnung ausgewählt wird, indem für einen vorgegebenen Träger, der eine selektive Schicht mit einem vorgegebenen Brechungsindex als dünne Schicht aufweist, und für eine vorgegebene Deckschicht die Dicke der selektiven Schicht variiert wird, für jede Schichtdicke der Reflexionsgrad für verschiedene Wellenlängen berechnet und in einem Diagramm gegen die Schichtdicke in nm aufgetragen wird, für jede Wellenlänge die Ableitung des Reflexionsgrades nach der Schichtdicke gebildet wird, und für eine gegebene Schichtdicke die optimale Wellenlänge als die Wellenlänge bestimmt wird, bei der die Ableitung bei der gegebenen Schichtdicke ein Extremum hat.

2. Verfahren nach Anspruch 1, **dadurch gekennzeichnet, daß** der reflektierte Strahlenanteil in Richtung einer maximalen Änderung der detektierten Lichtintensität durch Variieren des Einfallswinkels und der Polarisationsrichtung des Lichts optimiert wird.

3. Verfahren nach einem der Ansprüche 1 bis 2, **dadurch gekennzeichnet, daß** für die Untersuchung ein Träger aus Kunststoff zur Aufnahme der dünnen Schicht verwendet wird.

4. Verfahren nach einem der Ansprüche 1 bis 3, **dadurch gekennzeichnet, daß** für das Licht einer einzigen Wellenlänge eine Lichtquelle verwendet wird, die monochromatisches Licht ausstrahlt.

5. Verfahren nach Anspruch 4, **dadurch gekennzeichnet, daß** als monochromatische Lichtquelle eine Leuchtdiode, Laserdiode oder ein Laser verwendet wird.

6. Verfahren nach einem der Ansprüche 1 bis 3, **dadurch gekennzeichnet, daß** für das Licht einer einzigen Wellenlänge eine polychromatische Lichtquelle verwendet wird, der mindestens ein Monochromator nachgeordnet wird.

7. Verfahren nach Anspruch 6, **dadurch gekennzeichnet, daß** der zumindest eine Monochromator vor oder hinter der dünnen Schicht angeordnet wird.

8. Verfahren nach einem der Ansprüche 1 bis 7 zur Untersuchung von Antigen-Antikörper- und/oder DNA- DNA-Wechselwirkungen.

## Claims

1. A method of marker-free examination of biochemical reactions, bonding and/ or attachment processes on/or in at least one thin layer whose thickness is between 0,1 nm und 100 µm, preferably between 10 nm und 2.000 nm, comprising the steps of:
- illumination of at least one punctiform or area-measured region of the at least one thin layer with light of at least one light source (1),
- directing that part of the radiation reflected on consecutive boundary surfaces of the at least one thin layer to at least one detector (6),
- detecting of that part of the radiation reflected on consecutive boundary surfaces of the at least one thin layer with the at least one detector (6), the light incident to the at least one detector being monochromatic,
- measurement of the modification of the detected light intensity caused by the change of the optical layer thickness due to the biochemical reactions, bonding and/or attachment processes,
- deducting of parameters from the change of the detected light intensity which characterise the reactions, bonding and/or attachment processes on/or in the at least one thin layer,
**characterised in that** one single optimal wavelength is selected for the monochromatic light incident to the at least one detector, wherein
- the wavelength most suitable for the examination is selected via experimental measurements by recording a thin-layer spectrum of the thin layer, thereby plotting the light intensity as a function of the wavelength and determining the optimal wavelength in the range of a turning point of the thin-layer spectrum, or
- the wavelength most suitable for the examination is selected via theoretical calculation by varying the thickness of a selective layer for a given cover layer and for a given support showing the selective layer with a given refractive index as thin layer, calculating the degree of reflexion for different wavelengths for each layer thickness of the selective layer, plotting it in a diagram against the layer thickness in nm, calculating the derivative of the degree of reflexion with respect to the layer thickness for each wavelength, and determining the optimal wavelength for a given layer thickness as being the wavelength for which by the given layer thickness the derivation has an extremum.

2. The method according to claim 1, **characterised in that** the reflected part of the radiation is optimised by varying the angle of incidence and the light's polarisation direction towards a maximum change of the detected light intensity.

3. The method according to any of the claims 1 to 2, **characterised in that** for the purpose of the examination a carrier made of plastic is used for receiving the thin layer.

4. The method according to any of the claims 1 to 3, **characterised in that** for the light of one single wavelength a light source is used that emits monochromatic light.

5. The method according to claim 4, **characterised in that** a light emitting diode, a laser diode or a laser is used as the monochromatic light source.

6. The method according to any of the claims 1 to 3, **characterised in that** a polychromatic light source is used for the light of one single wavelength, whereby the polychromatic light source is followed by at least one monochromator.

7. The method according to claim 6, **characterised in that** the at least one monochromator is arranged in front of or subsequent to the thin layer.

8. The method according to any of the claims 1 to 7 for the examination of antigen-antibody interactions and/or DNA- DNA interactions.

## Revendications

1. Procédé d'examen sans marqueur de réactions biochimiques, de mécanismes de liaison et/ou d'addition sur ou dans au moins une couche mince, dont l'épaisseur est comprise entre 0,1 nm et 100 µm, de préférence entre 10 nm et 2 000 nm, comprenant les étapes suivantes :
- une illumination d'au moins une zone ponctuelle ou plane de la au moins une couche mince avec de la lumière provenant d'au moins une source de lumière (1),
- une transmission de la partie de rayons réfléchie au niveau des périphéries successives de la au moins une couche mince à au moins un détecteur (6),
- une détection de la partie de rayons réfléchie au niveau des périphéries successives de la au moins une couche mince à l'aide du au moins un détecteur (6), où la lumière reçue sur le au moins un détecteur est monochromatique,
- une mesure du changement de l'intensité lumineuse détectée provoqué par un changement de l'épaisseur de couche optique après les réactions biochimiques, les mécanismes de liaison et/ou d'addition,
- une dérivée de paramètres à partir du changement de l'intensité lumineuse détectée, qui caractérisent les réactions, les mécanismes de liaison et/ou d'addition à examiner sur et/ou dans la au moins une couche mince,
**caractérisé en ce que**, pour la lumière monochromatique reçue sur le au moins un détecteur, une seule longueur d'onde optimale est choisie, où
- la longueur d'onde optimale pour l'examen est choisie par le biais de mesures expérimentales, en établissant un spectre de couche mince de la couche mince, selon lequel l'intensité lumineuse est présentée comme une fonction de la longueur d'onde et en déterminant la longueur d'onde optimale dans la zone d'un point d'inflexion du spectre de couche mince, ou
- la longueur d'onde optimale pour l'examen est choisie par le biais d'un calcul théorique, en prenant en compte, pour un support prédéfini, la une couche sélective avec un indice de réfraction prédéfini en tant que couche mince, et en faisant varier, pour une couche de surface prédéfinie, l'épaisseur de la couche sélective, en calculant, pour chaque épaisseur de couche, le facteur de réflexion pour les différentes longueurs d'onde et en le représentant dans un diagramme en fonction de l'épaisseur de couche en nm, en traçant, pour chaque longueur d'onde, la dérivée du facteur de réflexion en fonction de l'épaisseur de couche, et en déterminant, pour une épaisseur de couche donnée, la longueur d'onde optimale comme étant la longueur d'onde, pour laquelle la dérivée pour l'épaisseur de couche donnée présente un extremum.

2. Procédé selon la revendication 1, **caractérisé en ce que** la partie de rayons réfléchie est optimisée pour un changement maximal de l'intensité lumineuse détectée par le biais de modifications de l'angle d'incidence et de la direction de polarisation de la lumière.

3. Procédé selon l'une des revendications 1 à 2, **caractérisé en ce que** pour l'examen un support en matière synthétique pour la réception de la couche mince est employé.

4. Procédé selon l'une des revendications 1 à 3, **caractérisé en ce que** pour la lumière, une seule longueur d'onde d'une source de lumière est employée, laquelle émet de la lumière monochromatique.

5. Procédé selon la revendication 4, **caractérisé en ce qu'**une diode électroluminescente, une diode laser ou un laser est employé en tant que source de lumière monochromatique.

6. Procédé selon l'une des revendications 1 à 3, **caractérisé en ce que** pour la lumière, une seule longueur d'onde d'une source de lumière polychromatique est employée, derrière laquelle au moins un monochromateur est agencé.

7. Procédé selon la revendication 6, **caractérisé en ce que** le au moins un monochromateur est agencé devant ou derrière la couche mince.

8. Procédé selon l'une des revendications 1 à 7 pour un examen des interactions antigène-anticorps et/ou ADN-ADN.
